# EUROPEAN PATENT SPECIFICATION

(11) **EP 2 617 703 B1**
(45) Date of publication and mention of the grant of the patent: **30.07.2014**
(21) Application number: 12305062.7
(22) Date of filing: 17.01.2012
(51) Int. Cl.: C07C 45/30, C07C 47/54, C07C 47/02, C07C 49/403, C07B 33/00, C07B 41/06

(54) **Improved catalyzed hypohalous oxidation of alcohol groups**
Verbesserte katalysierte unterhalogenige Oxidierung von Alkoholgruppen
Oxydation hypohalogéneuse catalysée améliorée de groupes d'alcool

(43) Date of publication of application: 24.07.2013
(73) Proprietor: Corning Incorporated, Corning NY 14831 (US)
(72) Inventor: Horn, Clemens, 91630 GUIBEVILLE (FR); Gaurat, Olivier, 77140 NEMOURS (FR); Jean, Patrick, 77820 LE CHATELET EN BRIE (FR)
(74) Representative: Le Roux, Martine

(56) References cited:
- PHILIP D. HAMPTON ET AL: "Continuous Organic Synthesis in a Spinning Tube-in-Tube Reactor: TEMPO-Catalyzed Oxidation of Alcohols by Hypochlorite", ORGANIC PROCESS RESEARCH & DEVELOPMENT, vol. 12, no. 5, 19 September 2008 (2008-09-19), pages 946-949, XP55026896, ISSN: 1083-6160, DOI: 10.1021/op800051t
- W. FERSTL ET AL.: "Process optimization of a catalyzed bleach oxidation for the production of functionalized aldehydes using microreaction technology", CHEM. ENG. J., vol. 1355, 2008, pages S292-S297, XP002676315, ISSN: 1385-8947

## Description

### FIELD OF THE INVENTION

The present invention concerns a process for oxidation of alcohols to aldehydes and ketones. It more particularly concerns an improved process for oxidation of at least one alcohol group of at least one chemical compound to the corresponding carbonyl group, in the presence of a buffered oxidative hypohalous solution and a nitroxide oxidation catalyst, such as TEMPO ((2,2,6,6-tetramethylpiperidin-1-yl)oxyl).

### BACKGROUND OF THE INVENTION

The TEMPO oxidation of alcohols to aldehydes and ketones was discovered in 1987 (1). According to (1), the reaction is batchwise performed, at a lab scale, with freshly prepared bleach as oxidant (the problem of the degradation of bleach solutions (more and more critical as their pH decreases below 9.5) being well-known from the man skill in the art). It is more particularly performed at 0°C, at a pH of 8.6, with 1 mol % of TEMPO and 10 mol % of co-catalyst like KBr.

Since 1987 the TEMPO oxidation with a hypohalous solution became more and more popular replacing other oxidations like for example PCC (Pyridine ChloroChromate) oxidation. In 2011, nearly 20,000 reactions using TEMPO-type oxidations (carried out with hypochlorous solutions) have been published. The reaction conditions have not much changed since 1987 and referred as standard (2, 3). Other catalysts have been developed, notably polymer-bonded derivatives like PIPO (Polymer Immobilized TEMPO) (4). The use of immobilized TEMPO in batch reactors and in micro-channels was respectively mentioned in (4) and in European patent application (5). US patent (6) describes the use of TEMPO in range of 0.01 to 100 mol %, but preferably 0.1-2 mol %, in a context to generate a carbonyl group in organosilicon compound. Meanwhile the TEMPO oxidation is considered as "part of the central tool box in the industrial organic chemistry" (7).

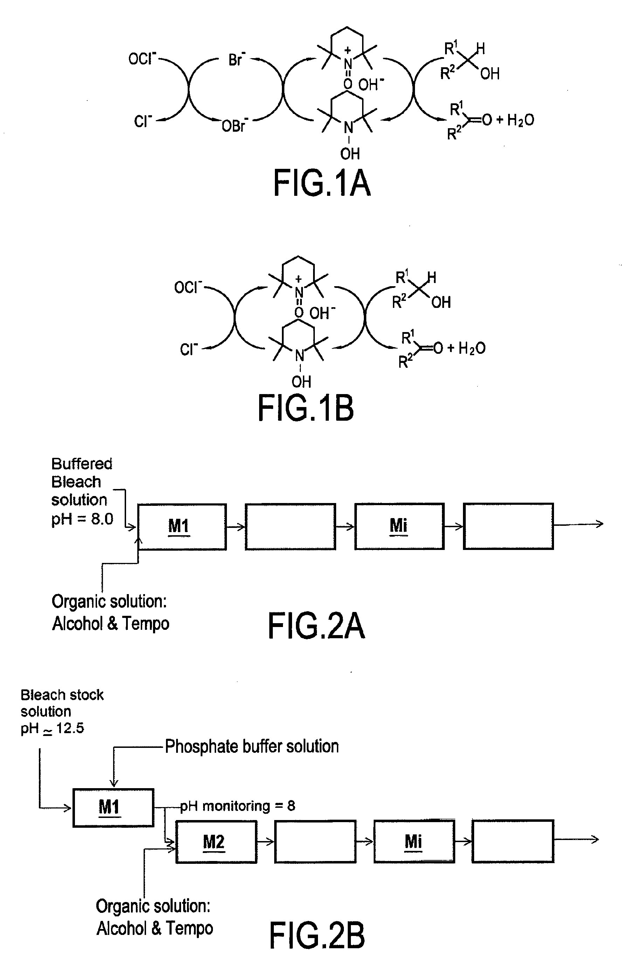

The proposed and commonly accepted mechanism (see attached Figure 1A) suggests that the use of the co-catalyst accelerates the reaction. The reaction can work without co-catalyst but might take much longer even when the temperature is increased to room temperature.

When carrying out the TEMPO reaction (a highly exothermic reaction) batchwise at a higher scale, the bleach is slowly added and the stability of said bleach is a more critical problem. So (8) discloses carrying out said reaction at a pH of 9.5 (with the co-catalyst KBr).

Carrying out the TEMPO reaction in a micro-reactor or tube reactor is also known, applying the above mentioned conditions or even more catalyst (9-11). E. Fritz-Lanhghals specially showed the industrial feasibility of this reaction in a tube reactor, with 25 sec residence time at a pH of 9.5 using 2 mol % of catalyst and 2 mol % of co-catalyst (11). The TEMPO-catalysed oxidation of alcohols by hypochlorite has also been carried out in a spinning tube-in-tube reactor (12). The conditions of the reaction are very similar to the ones disclosed in (1). The bleach solution (commercial solution saturated in sodium bicarbonate) is freshly prepared, a co-catalyst is used: Bu₄NBr, which acts both as co-catalyst (Br⁻) and as phase-transfer agent (Bu₄N⁺). In view of the given results, the presence of Bu₄NBr appears to be a key point. The reaction is carried out at a lab scale and requires energy to spin the rotor of the reactor. No teaching in reference to the problem of the management of the feedstock of the oxidant solution (not stable at the indicated pH) is disclosed.

From a process point of view, alternative methods for continuous flow oxidation of alcohols and aldehydes (alternatives to the TEMPO oxidation) have been proposed:
- the method disclosed in (13) utilizing bleach and catalytic tetrabutylammonium bromide. The reaction conditions are mild and generally provide complete conversion in 5-30 min;
- the method disclosed in (14) based on the elimination of H₂ in the presence of a copper catalyst in gas/liquid flow.

In such a context, the inventors propose an improved way of carrying out a TEMPO-type oxidation. They actually have determined conditions under which the action of the oxidative solution is optimized.

### THE INVENTION

The improved TEMPO-type oxidation process of the invention is hereafter described in detail.

A process for oxidation of at least one alcohol group (= at least one hydroxyl group) of at least one chemical compound to the corresponding carbonyl group is more generally concerned.

Said process can be carried out for the oxidation of a single chemical compound (with alcohol group(s) in its formula) or for the oxidation of (a mixture of) at least two chemical compounds (with alcohol group(s) in their formulae). It is generally carried out for the oxidation of a single chemical compound.

It has here to be indicated that the concerned chemical compound(s) with at least one alcohol group to oxidize according to the process of the invention is(are) quite able to include, in addition to said at least one alcohol group to oxidize, at least another alcohol group, which is protected from oxidation and consequently not concerned by the oxidation process of the invention.

In its(their) chemical formula(e), the concerned compound(s) has(have) at least one alcohol group (= at least one hydroxyl) group. It (they) actually has(have) at least one primary alcohol group (a single one or at least two), or at least one secondary alcohol group (a single one or at least two), or at least one primary alcohol group and at least one secondary alcohol group (in that later case, the concerned chemical compound(s) has(have) both primary alcohol group(s) and secondary alcohol group(s) in its(their) chemical formula(e)). It is here indicated that, in case of the presence of at least two alcohol groups to oxidize according to the invention, it can sometimes be possible to carry out a selective oxidation, thanks to a setting of the residence time in the concerned reactor. So, for example, if primary and secondary alcohol groups are present, the process of the invention can be carried out to oxidize both the primary and secondary alcohol groups or to oxidize only the primary alcohol group(s). This will be recalled later in the present text.

The man skilled in the art knows that a primary alcohol group is oxidized to an aldehyde group while a secondary alcohol group is oxidized to a ketone group.

So the process of the invention is suitable for the oxidation of a primary alcohol to an aldehyde and of a secondary alcohol to a ketone. It is more generally suitable to oxidize primary alcohol group(s) or secondary alcohol group(s) or both primary and secondary alcohol group(s) of a chemical compound or of a mixture of chemical compounds. The oxidation in question can be a selective one.

The oxidation process of the invention is conventionally carried out in the presence of a buffered oxidative hypohalous solution (such as a bleach solution) and of a nitroxide oxidation catalyst (such as TEMPO). It conventionally takes place in a biphasic system. The used oxidant and catalyst are those of the prior art.

Characteristically, the process for oxidation (of at least one alcohol group of at least one chemical compound to the corresponding carbonyl group) of the invention is carried out:
- within a micro-reactor (see below), and
- with the buffered oxidative hypohalous solution extemporaneously (see below) prepared at a buffered pH comprised between 7 and less than 8.5 (7 ≤ pH < 8.5).

The inventors have shown that it is possible, using an extemporaneously prepared buffered oxidative solution and carrying out the reaction in a micro-reactor, to manage the oxidation reaction in pH conditions under which said buffered oxidative solution is known as being not stable and that then very interesting results are obtained: maximum yields reached in a very short time (generally ≤ 3 min, indeed ≤ 1 min 30 s), i.e. a high rate of the oxidation reaction, and that without the use of any co-catalyst and at room temperature, indeed with the use of low quantity of catalyst (see further). In other words, the instability zone of the oxidative solution is a very favorable zone to carry out the reaction and it is possible to have the degradation rate of the oxidative solution slower than the reaction rate. Such a result was hard to foresee.

Characteristically, the process of the invention is carried out inside a micro-reactor. Here is a key of said process. It is so carried out in a continuous way inside a micro-reactor. Micro-reactors are reactors well-known from the man skilled in the art (15). They can be made from different materials such as stainless steels, plastics, glasses, glass-ceramics and ceramics. Micro-reactors can be obtained from different processes, more particularly from those described by the applicant in patent applications US 2004/0206391 and WO 2008/106160. Micro-reactors are more and more used today. Their reactant passages showing a circular or non-circular section generally have a hydraulic diameter within the following range: 0.2 - 15 mm. The process of the invention is carried out within such a micro-reactor, it is advantageously carried out within a micro-reactor with reactant passage(s) showing a circular or non-circular section having a hydraulic diameter within the following range: 0.2 - 4 mm. The process of the invention is obviously carried out in a micro-reactor, the constitutive material of which (or at least the material aimed to be in contact with the reactive medium of which) is compatible with the reactive medium. It is advantageously carried out in a micro-reactor made of glass, glass-ceramic or ceramic.

Characteristically, the process of the invention is carried out using a buffered oxidative hypohalous solution extemporaneously prepared at a buffered pH comprised between 7 and less than 8.5 (7 ≤ pH < 8.5), as the oxidant.

The pH value of the buffered oxidative solution is another key of the process of the invention. It is comprised between 7 and less than 8.5.

Said pH value is advantageously comprised between 7.5 and 8.4 (7.5 ≤ pH ≤ 8.4).

It is very advantageously comprised between 7.8 and 8.2 (7.8 ≤ pH ≤ 8.2). According to a particularly preferred embodiment, it is equal to 8. These very advantageous and particularly preferred ranges of the pH value are more particularly indicated in reference to the oxidation of primary alcohol group(s).

For the oxidation of secondary alcohol group(s), the pH value of the buffered oxidative hypohalous solution is generally advantageously comprised between 8 and 8.4 (8 ≤ pH ≤ 8.4).

The pH of the oxidative buffered hypohalous solution can have been adjusted to a desired value within the given range: 7 - less than 8.5 thanks to any method well known from the man skilled in the art.

The oxidative hypohalous solution used, which is buffered at a pH within the above indicated pH range, is extemporaneously prepared. Here is the last key of the process of the invention. The extemporaneously preparation of the buffered oxidative hypohalous solution is required in view of the instability of said solution at said pH. The life time of the hypohalous solution in the pH range: 7 - less than 8.5 is actually limited. Thus, the prepared oxidative hypohalous solution has to be rapidly used (has to be rapidly reacting with the compound(s) having the alcohol group(s) in the presence of the catalyst). It has to be used in less than 10 minutes after its preparation (= "extemporaneously prepared"). Advantageously, the prepared solution is used in less than 5 minutes and very advantageously in less than 3 minutes. All the used oxidative hypohalous solution has actually to be freshly prepared. It is particularly preferred to carry out the reaction as said oxidative solution is being prepared (see below).

According to a first variant, the buffered oxidative hypohalous solution is extemporaneously batchwise prepared outside the micro-reactor before its introduction into said micro-reactor. A buffering of a hypohalous solution to a desired pH value before (just before) its use can so be carried out. The prepared amount of the buffered oxidative hypohalous solution advantageously corresponds to the rapidly used amount of the buffered oxidative hypohalous solution. Said first variant is more particularly suitable for carrying out the process of the invention for small scale applications, more particularly in a laboratory.

According to a second variant, which is preferred, the buffered oxidative hypohalous solution is extemporaneously in-line prepared. A hypohalous solution and a buffer solution can so be mixed just before the oxidation reaction in a mixing unit arranged upwardly from the micro-reactor. It is quite possible for such a mixing unit to consist in a module of micro-reactor. In such a case the micro-reactor used for carrying out the process of the invention comprises a first module constituting a mixing unit (for the extemporaneous preparation of the buffered oxidative hypohalous solution) and at least one additional module for carrying out the oxidation reaction. The pH of the in-line buffered hypohalous solution can also be monitored on-line. Said second variant (according to which the oxidative solution is used as it being prepared) is highly advised when the reaction is carried out on an industrial scale (i.e. with large reaction volumes).

Here above have been indicated the three combined keys of the process of the invention: carried out in a **micro-reactor,** with **the buffered oxidative hypohalous solution extemporaneously prepared, at a buffered pH comprised between 7 and less than 8.5.**

Concerning the required amount of catalyst, the following may be indicated in a non limitative way. The conventional catalyst (nitroxide oxidation catalyst, see above) is generally used in less than 5 mol %, advantageously less than 2 mol %, very advantageously less than 0.5 mol %, in the process of the invention (mol % = percentage of mol equivalent referred to the compound(s) present to oxidize).

It has to be noted that the process of the invention for the oxidation of primary alcohol group(s), surprisingly requires less catalyst than the process of the prior art and that it can so be carried out with a concentration of catalyst less than 2 mol %, advantageously less than 0.5 mol %, very advantageously less than 0.07 mol %. Very good results have been obtained with the catalyst present at 0.05 mol %.

Considering now the co-catalyst (generally a source of Br⁻, such as KBr, Br₂, NaBr, R₄NBr (R being an alkyl group, for example), KBr being widely used) generally used in the prior art TEMPO-type oxidations of alcohols (see above as well as the attached figure 1A), it is not totally excluded to use it in carrying out the process of the invention. However the inventors have surprisingly noted that the oxidation process of the invention does not require its presence. The addition of a co-catalyst can actually be quite useless in view of the good results obtained without it. So the process of the invention is generally carried out without any co-catalyst (see the attached figure 1B). That is particularly interesting, not only in reference to the realized savings but also in reference to the byproducts able to be obtained when a co-catalyst is present (any halogen exchange with bromide (co-catalyst), when halogenated materials are involved, is avoided).

In the same way, it is not totally excluded to use a phase-transfer agent in carrying out the process of the invention. However the presence of such a phase-transfer agent is in no way compulsory.

It has been here above specified that the oxidation process of the invention is carried out in the presence of a buffered oxidative hypohalous solution, such an oxidative buffered hypohalous solution being known in the prior art. Such an oxidative buffered hypohalous solution advantageously consists in a bleach solution.

It has been here above specified that the oxidation process of the invention is carried out in the presence of a nitroxide oxidation catalyst, such a catalyst being known in the prior art. So such a nitroxide oxidation catalyst is advantageously selected, for carrying out the process of the invention, from the group consisting in the known nitroxide oxidation catalysts, from the group consisting in the (2,2,6,6-tetramethylpiperidin-1-yl)oxyl (TEMPO), in the TEMPO derivatives (such as HOT (4-hydroxy-TEMPO), 4-acetamido-TEMPO) and in the TEMPO or TEMPO derivatives on a support (such as PIPO (Polymer immobilized TEMPO)).

The used nitroxide oxidation catalyst is preferably TEMPO.

The process of the invention is advantageously carried out with the buffered oxidative hypohalous solution as identified above and/or (very advantageously and) with the nitroxide oxidation catalyst as indentified above.

The process of the invention is perfectly efficient at room temperature (between 15 and 25°C). It is generally carried out at about 20°C (between 18 and 22°C).

The process according to the invention can be carried out with or without solvent. It more particularly depends on the nature of the chemical compound(s) with the alcohol group(s) concerned.

The used solvent (if any) is advantageously selected from the group consisting in:
- the alkanes such as pentane, hexane, heptane, octane, cyclohexane,
- the aromatic solvents such as benzene, toluene, xylene,
- the esters such as ethyl acetate, isopropyl acetate,
- the chlorinated solvents such as dichloromethane, chloroform, carbon tetrachloride, ethylene tetrachloride,
- the ketones such as acetone, butanone, pentanone, methyl isopropyl ketone, methyl isobutyl ketone, and
- their mixtures.

The used solvent is very advantageously selected from the environmental friendly solvents.

Carrying out the conventional TEMPO-type oxidation in the above described conditions of reaction of the invention allows obtaining the maximum yield (the yield is a notion familiar to the man skilled in the art. It is expressed in % as a ratio (in mass or in mole) of the aimed obtained compound/theoretically aimed obtained compound and the maximum yield is also a notion familiar to the man skilled in the art. The reaction being complete, the yield reaches a maximum value and is not able to increase even if the experimental conditions are longer maintained (and is even able to decrease if the obtained compound is not stable)) very rapidly, allows the rate of the TEMPO-type oxidation to be surprisingly high. In a non-limitative way, it can here be indicated that the maximum yield value(s) is(are) generally of at least 50%, is(are) advantageously of at least 80% and that said maximum yield value(s) is(are) reached in a very short time (generally ≤ 3 min, indeed ≤ 1 min 30 s) and that at room temperature, without the use of any co-catalyst.

The chemical compound or at least one of the chemical compounds, the alcohol group of which or at least one of the alcohol groups of which has to be oxidized according to the process of the invention, can be injected within the micro-reactor at a single injection point (generally at the entrance of the reactor) or at at least two injection points (a first one and at least one additional one), at least one of said at least two injection points being positioned downstream from a first injection point (generally positioned at the entrance of the reactor). Such an injection v*ia* at least two injection points is advantageously carried out to improve the control of the temperature of the reaction medium.

As indicated above, micro-reactors are known *per se.* However, the man skilled in the art did not expect the so good result obtained according to the invention in using them in a context of alcohol group(s) oxidation.

According to an advantageous variant, micro-reactors suitable for implementing the process of the invention have at least two mixing zones along their reactant passage.

Such micro-reactors very advantageously have, successively along its length, at least two inlets, at least one for the buffered oxidative hypohalous solution, and at least one for the chemical compound(s) and TEMPO containing solution, an initial mixer passage portion, an initial dwell time passage portion having a volume of at least 0.1 ml and one or more additional mixer passage portions, each additional mixer passage portion followed immediately by a corresponding respective additional dwell time passage portion.

Within such micro-reactor, the reactants are mixed in a first mixing zone, immediately after their injection. Then, they go through a dwell time passage portion. The sequence: mixing zone/dwell time zone is at least repeated twice. So, two mixings are at least carried out within such a micro-reactor. Such a micro-reactor is recommended for carrying out the process of the invention.

Such micro-reactors have been described in European patent application (16) by the applicant.

According to a second advantageous variant, micro-reactors suitable for implementing the process of the invention are micro-reactors which ensure a permanent mixing all along their reactant passage.

Such micro-reactors very advantageously have a reactant passage which comprises multiple successive chambers, each chamber including a split of the reactant passage into at least two sub-passages, and a joining of the split passages, and a change of passage direction, of at least one of the sub-passages, of at least 90 degrees. According to a preferred variant, each of the multiple successive chambers, being immediately succeeded by another one of said chambers, further comprises a gradually narrowing exit which forms a corresponding narrowed entrance of the succeeding chamber and a splitting and re-directing wall oriented crossways to the immediately upstream passage and positioned immediately downstream of the chamber's entrance, the upstream side of said splitting and re-directing wall having a concave surface.

Within such micro-reactors, the mixing is permanent.

Such micro-reactors have been described in European patent application (17) by the applicant.

The man skilled in the art knows how to optimize the compromise: efficiency of mixing / pressure drop in determining the structure of a micro-reactor. So, he will know how to optimize said compromise in determining the structure of the micro-reactors, used for carrying out the process of the invention.

As explained above, the process of the invention is able to be carried out in many contexts. It can be carried out to oxidize any (unprotected) alcohol group of the chemical compound(s) present in the reaction medium. It can be carried out to implement a selective oxidation. A selective oxidation is able to be managed by the setting of the residence time inside the reactor, supposing that there exists a sufficient difference of reactivity between some different alcohol groups (such a difference of reactivity being for example based on different steric hindrances and/or on a different chemical environment).

According to a preferred variant, the oxidation process of the invention is carried out for oxidation of the alcohol group or all the alcohol groups of a single chemical compound. Within the scope of said preferred variant, it is advantageously carried out for oxidation of a single alcohol group of a single chemical compound; it is very advantageously carried out for oxidation of a single primary alcohol group of a single chemical compound (so as to generate an aldehyde; the oxidation of the 1-octanol and equivalents being an example).

According to another preferred variant, the oxidation process of the invention is carried out for a selective oxidation of at least one alcohol group within a mixture comprising at least two chemical compounds, each having at least one alcohol group or of at least one alcohol group, of a single chemical compound.

Within the scope of said preferred variant, it can be carried out for the selective oxidation of primary alcohol group(s) within a mixture of at least two chemical compounds, each having at least one alcohol group, or it can be carried out for the selective oxidation of primary alcohol group(s) of a chemical compound having said primary alcohol group(s) and at least one secondary alcohol group (the primary alcohol group(s) is(are) oxidized while the secondary alcohol group(s) is(are) not); it can be carried out for selective oxidation of at least one primary alcohol group of a chemical compound having primary alcohol groups (the selection is operated between the primary alcohol groups, in the presence or the absence of secondary alcohol group(s)) or for selective oxidation of at least one secondary alcohol group of a chemical compound having secondary alcohol groups (the selection is operated between the secondary alcohol groups, in the possible presence of aldehyde group(s) (resulting from the oxidation of at least a primary alcohol group of the chemical compound)).

No doubt that the man skilled in the art has already understood the great interest of the process of the invention. The main advantages of the process of the invention are hereafter summarized:
- the oxidation reaction goes rapidly to completion, generally in less than 3 minutes, sometimes within seconds,
- the process is efficient at room temperature and without co-catalyst,
- reduced amounts of catalyst can be used,
- the process can be carried out for selective oxidations,
- the process allows in-line monitoring of the pH to optimize the rate and yield of the reaction.

The claimed invention is now illustrated, in a non-limitative way, by the following examples and figures.

### FIGURES

Figure 1A (taken from (2)) shows the proposed reaction mechanism for a TEMPO oxidation of a secondary alcohol with KBr as co-catalyst.
Figure 1B schematically shows the proposed reaction mechanism for the same TEMPO oxidation performed according to the preferred variant of the process of the invention without any co-catalyst.
Figure 2A schematically shows the process of the invention (carried out in a micro-reactor without in-line pH adjustment).
Figure 2B schematically shows the process of the invention (carried out in another micro-reactor with in-line pH adjustment or control).
Figure 3 shows the impact of the initial pH of the extemporaneously prepared buffered bleach solution on the yields and conversion, for a TEMPO oxidation carried out according to the invention (see below example 1A).
Figure 4 shows the impact of the degradation of the (non-extemporaneously prepared) bleach solution on the yields and conversion for a TEMPO oxidation (see below example 1A').

These figures are self-explanatory and/or have been commented in the above specification and/or are commented in the below examples.

### • Examples A, B, BA,BB and BC (Prior Art)

### • Example A

The reaction is carried out in a round-bottomed flask with magnetic stirring (1000 rpm) at 0°C (ice bath). The reaction takes place in a biphasic system. Alcohol (octanol-1: 25 mmol, 3.94 ml, 3.25 g), TEMPO (1 mol%, 0.25 mmol, 39 mg) and an organic solvent (dichloromethane, 46 ml) are poured into the flask. **Co-catalyst KBr** (10 mol%, 2.5 mmol, 298 mg) **is added next.**

The solution of bleach is prepared at 5 °C. The stock solution of bleach (10% w/w; 1.5 eq.; 22.6 ml) is diluted up to 50 ml with water. Then NaHCO₃ is added stepwise (three spatulas) to obtained a saturated solution. The pH is near 9. The bleach solution is added to the dichloromethane solution under vigorous stirring. The reaction is stirred with ice bath cooling. Samples are taken every minute during 5 minutes and then every 5 minutes. The samples are quenched with sodium thiosulfate and analyzed by GC(Gas Chromatography).

### • Example B

The conditions are the same as in Example A but toluene is used instead of dichloromethane.

### • Example BA

The conditions are the same as in Example B **without co-catalyst KBr**.

### • Example BB

The conditions are the same as in Example BA. The reaction is performed at 20 °C

### • Example BC

The conditions are the same as in Example BB, but 0.05 mol% TEMPO is used.

The results are given in following Table 1.

**Table 1**

| Example | Yield (%) | Time (min) |
|---|---|---|
| A | >98 | 4 |
| B | >98 | 5 |
| BA | 66 (reaction not complete) | 20 |
| BB | >98 | 15 |
| BC | 96 | 144 |

### • Examples 1A, 1B, 1C (invention)

The micro-reactors used are glass micro-reactors with heart modules (said modules being schematically identified as Mi in figures 2A and 2B), as described in (17).

The volume of the micro-reactor used in example 1A is about 3.15 ml (≈ 0.45 ml (internal volume of each module Mi) x 7 (i=7)) + the additional volume of tubing. Its reactant passage has a diameter of about 0.38 mm.

The volume of the micro-reactor used in example 1B is about 3.6 ml (≈ 0.45 ml (internal volume of each module Mi) x 8 (i=8)) + the additional volume of tubing; one module (the first one (M1) being used for in-line constituting the buffered bleach solution and seven other modules for the reaction (M2 to M8)). So the reaction volume is about 3.15 ml. The reactant passage has also a diameter of about 0.38 mm.

The volume of the micro-reactor used in example 1C is about 56.2 ml (≈ 7.03 ml (internal volume of each module Mi) x 8 (i=8)) + the additional volume of tubing; one module (the first one (M1) being used for in-line constituting the buffered bleach solution and seven other modules for the reaction (M2 to M8)). So the reaction volume is about 49.2 ml. The reactant passage has a diameter between 0.95 and 1.25 mm. The modules of said micro-reactor are larger.

The feeding of the micro-reactors (more precisely, of the first module M1 of said micro-reactors) with the buffered bleaching solution is carried out according to two different ways:
- for example 1A : without in-line pH adjustment, as shown on Figure 2A and as explained in below example 1A;
- for examples 1B and 1C : with in-line pH adjustment, as shown on figure 2B (said figure schematically shows both the "small" modules of the micro-reactor used in example 1B and the larger modules of the micro-reactor used in example 1C) and as explained in below examples 1B and 1C.

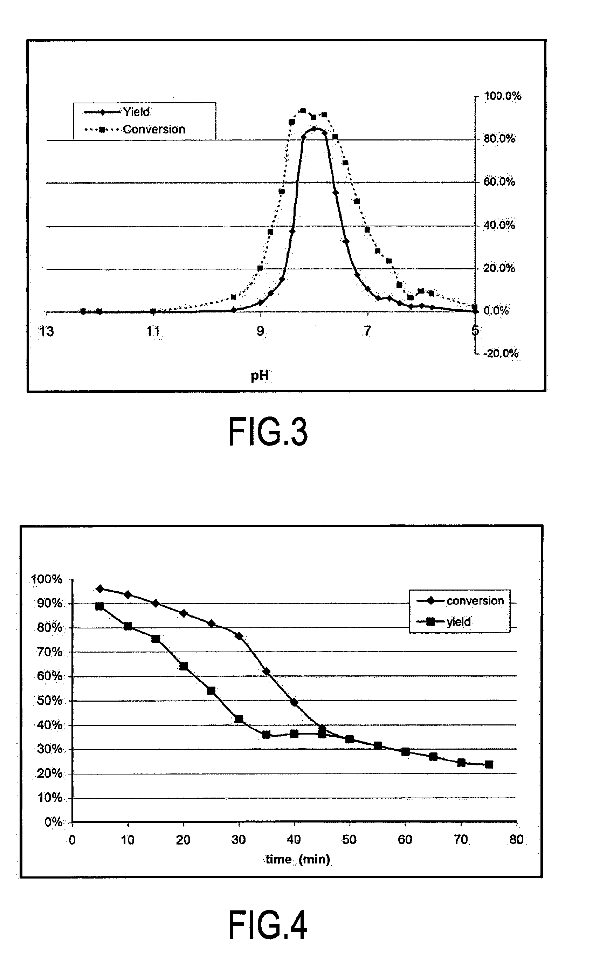

### Example 1A (figures 2A and 3) (including comparative data)

1-Octanol (c= 0.5 mol/l) and 0.05% (molar) of TEMPO are dissolved in toluene, so constituting a first solution (the organic solution shown on figure 2A).

For each trial, a small volume (about 50ml) of a second solution - solution of buffered bleach at a given pH (see below Table 2) - is extemporaneously prepared.

The two solutions are pumped into the micro-reactor (into the first module M1 of the micro-reactor).

The total flow rate is always divided up in approximately two equal parts between organic and aqueous phases (between the first and the second solutions). The residence time is about 16 s.

The temperature is 20°C.

The results are given in the following Table 2 and shown in annexed Figure 2. They demonstrate the critical character of the pH.

**Table 2**

| **pH** | **Yield octanal (%)** | **Conversion 1-octanol (%)** |
|---|---|---|
| 12.3 | 0.0 | 0.0 |
| 12 | 0.0 | 0.0 |
| 11 | 0.0 | 0.0 |
| 9.5 | 0.8 | 6.4 |
| 9 | 4.4 | 20.3 |
| 8.8 | 8.7 | 37.2 |
| 8.6 | 15.3 | 55.9 |
| **8.4** | **37.3** | **88.2** |
| **8.2** | **81.4** | **93.5** |
| **8** | **85.3** | **90.2** |
| **7.8** | **83.2** | **91.6** |
| **7.6** | **55.4** | **81.2** |
| **7.4** | **32.7** | **69.3** |
| **7.2** | **17.3** | **51.3** |
| **7** | **10.5** | **37.7** |
| 6.8 | 6.1 | 28.2 |
| 6.6 | 6.1 | 23.5 |
| 6.4 | 3.7 | 12.0 |
| 6.2 | 2.2 | 6.2 |
| 6 | 2.8 | 9.4 |
| 5.8 | 1.9 | 8.2 |
| 5 | 0.0 | 2.0 |
| 4.2 | 0.0 | 0.0 |
| 3.2 | 0.0 | 0.0 |

### Example 1B (figure 2B)

1-Octanol (c=1 mol/l) and 0.2 % (molar) of TEMPO are dissolved in dichloromethane, thus constituting a solution for a first feed (feed 1 = the organic solution shown on figure 2B)

A second feed (feed 2) is stock solution of bleach (c=11.2 %) at a pH about 12.5. A third feed (feed 3) is a 0.7 M phosphate buffer solution (pH 8). This solution is brought to pH 6.2 by slow and controlled addition of H₃PO₄ 85 wt. %. Feeds 2 and 3 (in a ratio buffer/bleach of 1.1) are mixed in one fluidic module (the first one M1 of the used micro-reactor) before passing a control of pH (=8) and the mixture (the aqueous phase) is then injected in the second module M2 of the micro-reactor.

Feed 1 is directly pumped to the module M2 where it is mixed with the aqueous phase.

The total ratio (aqueous/organic phase) is 1.33.

The temperature is 20°C.

The results obtained (conversion (%) and yield (%)) are indicated in the below Table 3 related to different experiments carried out at different residence time (Rt(s)).

**Table 3**

| **Conversion*** | **Yield**** | **Rt** |
|---|---|---|
| **(%)** | **(%)** | **(s)** |
| 100 | 87.8 | 73 |
| 98.7 | 90.6 | 57 |
| 92.2 | 84.1 | 49 |

| | | |
|---|---|---|
| * The conversion (%) is the ratio: difference between the starting octanol and the remaining octanol/the starting octanol. ** The yield (%) is the ratio: obtained aldhehyde /theoretically obtainable aldhehyde. | | |

The results (conversion (%) and yield (%)) clearly show the interest of the invention. The maximum yield is very rapidly obtained (in less than 80 s).

### Example 1C (figure 2B)

The micro-reactor used (of the type shown in figure 2B) has larger modules Mi than those used in examples 1A and 1B. It has been fed like the one of example 1B, as shown in figure 2B (two equal parts of the second and third feeds and two equal parts of the first and second solutions).

The two solutions are pumped into the micro-reactor. The total flow rate varies from 20 ml/min to 170 ml/min.

The temperature is 20°C.

The results obtained (conversion (%) and yield (%)) are indicated in the below Table 4 related to different experiments carried out at different flow rates (and consequently with different residence time (Rt(s)).

**Table 4**

| **Conversion** | **Yield** | **Rt** |
|---|---|---|
| **(%)** | **(%)** | **(s)** |
| 96% | 82% | 120,0 |
| 100% | 93% | 68,3 |
| 96% | 90% | 45,5 |
| 94% | 84% | 27,3 |
| 94% | 80% | 22,1 |
| 94% | 68% | 19,2 |
| 94% | 66% | 18,3 |
| 87% | 55% | 17,6 |

Said results clearly show the interest of the invention. The maximum yield is very rapidly obtained (in less than 80 s).

### Example 1A' (comparative example) (figures "2A" and 4)

The micro-reactor used is identical to the one of example 1A.

1-Octanol (c= 1 mol/l) and 0.2% (molar) of TEMPO are dissolved in dichloromethane, so constituting a first solution (the organic solution shown on figure 2A).

A large volume of a second solution - the one of buffered bleach - is not extemporaneously prepared (insofar as the trial lasts for 75 min) by mixing 1/1 volume of commercial bleach solution (10.6%, pH = 12.5) and 0.75 M of NaH₂PO₄ solution. The initial pH of this stock solution of bleach (1.2 equivalent) is adjusted to 8.16 by slow and controlled addition of H₃PO₄ 85 wt. %.

The two solutions are pumped into the micro-reactor (into the first module M1 of the micro-reactor).

The residence time is 73 s and the ratio between aqueous and organic phases (between the first and the second solutions) is 1.3.

The temperature is 20°C.

The samples are taken out every five minutes. The results obtained for conversion (%) and yield (%) are indicated in the below Table 5. The pH of the buffered bleach solution feed stock is also indicated to highlight its rapid evolution correlated to bleach instability.

**Table 5**

| **Time (min)** | **Conversion* (%)** | **Yield* (%)** | **pH** |
|---|---|---|---|
| 0 | | | 8.16 |
| 5 | 96.2 | 88.9 | 8.06 |
| 10 | 93.7 | 80.7 | 7.96 |
| 15 | 90.1 | 75.5 | 7.87 |
| 20 | 86.0 | 64.3 | 7.79 |
| 25 | 81.6 | 54.0 | 7.69 |
| 30 | 76.5 | 42.4 | 7.6 |
| 35 | 62.1 | 36.0 | 7.49 |
| 40 | 49.3 | 36.2 | 7.43 |
| 45 | 38.7 | 36.1 | 7.36 |
| 50 | 34.0 | 34.0 | 7.28 |
| 55 | 31.4 | 31.4 | 7.22 |
| 60 | 28.9 | 28.9 | 7.17 |
| 65 | 26.8 | 26.8 | 7.11 |
| 70 | 24.4 | 24.4 | 7.07 |
| 75 | 23.6 | 23.6 | 7.04 |

| | | | |
|---|---|---|---|
| * The conversion (%) is the ratio: difference between the starting 1-octanol and the remaining 1-octanol/the starting 1-octanol. ** The yield (%) is the ratio: obtained aldhehyde /theoretically obtainable aldhehyde. | | | |

The results (conversion (%) and yields (%)) are also shown in annexed Figure 4.

The curves of figure 4 clearly show high yield and conversion only at the beginning of the experiment followed by a rapid degradation of the (non-extemporaneously) prepared bleach solution. Said degradation (monitored *via* the pH of the feed stock (see table 5)) reduced drastically the yield and conversion after few minutes of run.

### Example 2 (figure 2B)

The micro-reactor used is identical to the one of example 1B.

Benzyl alcohol (c=1 mol/l) and 0.2 % (molar) of TEMPO are dissolved in dichloromethane, thus constituting a solution for a first feed (feed 1 = the organic solution shown on figure 2B).

A second feed (feed 2) is stock solution of bleach (c=11.2 %) at a pH about 12.5. A third feed (feed 3) is a 0.7 M phosphate buffer (pH 8). This solution is brought to pH 6.2 by slow and controlled addition of H₃PO₄ 85 % wt. Feeds 2 and 3 (in a ratio buffer/bleach of 1.1) are mixed in one fluidic module (the first one M1 of the used microreactor) before passing a control of pH (= 8) and the mixture is then injected in the second module M2 of the micro-reactor. Feed 1 is directly pumped to the module M2 where it is mixed with aqueous phase.

The total ratio (aqueous/organic phase) is 1.33.

The temperature is 20°C.

The results obtained (conversion (%) and yield (%)) are indicated in the Table 6 below related to different experiments carried out at different residence time (Rt(s)).

**Table 6**

| **Rt** | **Conversion*** | **Yield**** |
|---|---|---|
| **(s)** | **(%)** | **(%)** |
| 73 | 100 | 100 |
| 57 | 100 | 100 |
| 49 | 100 | 100 |

| | | |
|---|---|---|
| * The conversion (%) is the ratio: difference between the starting benzyl alcohol and the remaining benzyl alcohol/the starting benzyl alcohol. ** The yield (%) is the ratio: obtained aldhehyde /theoretically obtainable aldhehyde. | | |

### Example 3 (figure 2B)

The micro-reactor used is identical to the one of example 1B.

Cyclohexanol (c=1 mol/l) and 4 % (molar) of TEMPO are dissolved in dichloromethane, thus constituting a solution for a first feed (feed 1 = the organic solution shown on figure 2B).

A second feed (feed 2) is stock solution of bleach (c= 10%) at a pH about 12.5. A third feed (feed 3) is a 0.85 M phosphate buffer solution (pH 8). This solution is brought to pH 6.5 by slow and controlled addition of H3PO4 85 % wt. Feeds 2 and 3 (in a ratio buffer/bleach of 0.8) are mixed in one fluidic module (the first one M1 of the used micro-reactor) before passing a control of pH (=8) and the mixture is then injected in the second module M2 of the micro-reactor. Feed 1 is directly pumped to the module M2 where it is mixed with aqueous phase.

The total ratio (aqueous/organic phase) is 1.33.

The temperature is 20°C.

The results obtained (conversion (%) and yield (%)) are indicated in the below Table 7 related to different experiments carried out at different residence time (Rt(s)).

**Table 7**

| **Rt** | **Conversion*** | **Yietd**** |
|---|---|---|
| **(s)** | **(%)** | **(%)** |
| 73 | 96.1 | 93.3 |
| 57 | 94.3 | 94.3 |
| 49 | 92.8 | 92.8 |
| 38 | 91.5 | 91.5 |

| | | |
|---|---|---|
| * The conversion (%) is the ratio: difference between the starting cyclohexanol and the remaining cyclohexanol/the starting cyclohexanol. ** The yield (%) is the ratio: obtained ketone /theoretically obtainable ketone. | | |

### Example 4 (figure 2B)

The micro-reactor used is identical to the one of example 1B.

1-Octanol (c=0.5 mol/l), 2-Octanol (c=0.5 mol/l) and 0.2 % (molar) of TEMPO are dissolved in dichloromethane, thus constituting a solution for the first feed (feed 1 = the organic solution shown).

A second feed (feed 2) is stock solution of bleach (c=10.6 %) at a pH about 12.5. A third feed (feed 3) is a 0.75 M phosphate buffer solution (pH 8). This solution is brought to pH 6.3 by slow and controlled addition of H₃PO₄ 85 wt. %. Feeds 2 and 3 (in a ratio buffer/bleach of 1) are mixed in one fluidic module (the first one M1 of the used micro-reactor) before passing a control of pH (=8) and the mixture is then injected in the second module M2 of the micro-reactor. Feed 1 is directly pumped to the module M2 where it is mixed with the aqueous phase.

The total ratio (aqueous/organic phase) is 1.33.

The temperature is 20°C.

The results obtained (conversion (%) and yield (%)) are indicated in the below Table 8 related to an experiment carried out at 49 s of residence time (Rt(s)).

**Table 8**

| **Conversion* 1-octanol (%)** | **Yield 1-octanol (%)** | **Conversion* 2-octanol (%)** | **Yield 2-octanol (%)** | **Rt (s)** |
|---|---|---|---|---|
| 98.5 | 86.4 | 28 | 28 | 49 |

| | | | | |
|---|---|---|---|---|
| * The conversion (%) is the ratio: difference between the starting alcohol and the remaining alcohol /the starting alcohol. ** The yield (%) is the ratio: obtained aldhehyde /theoretically obtainable aldhehyde. | | | | |

This non-optimized experiment shows that it will be possible to carry out a selective oxidation with a shorter residence time.

### REFERENCES

(1) P.L. Anelli et al. J. Org. Chem 1987, 52, 2559-2562
(2) R. Sheldon in Acc. Chem. Res 2002, 35, 774
(3) For example, WO-A-2010/023678
(4) For example, US 6 995 291
(5) EP-A-1 534 421
(6) US 7 700 196
(7) R. Ciriminna et al. Org. Proc. Res. Dev. 2010, 14, 245
(8) P.L. Anelli et al, Org. Synth. 1990, 69, 212-219
(9) A. Bodgan et al. Beilstein J. Org. Chem. 2009, 5, 17
(10) W. Ferstl et al. Chem. Eng. J. 2008, 135S, S292
(11) E. Fritz-Langhals Org. Proc. Res Dev. 2005, 9, 577
(12) P. D. Hampton et al Org. Proc Res. Dev. 2008, 12, 946-949
(13) A.B. Leduc et al. dx.doi.org/10.1021/op200118h Org. Proc. Res. Dev. 2012, XXX, 000-000
(14) US 7 795 475
(15) N. Kockmann et al. Micro Process Engineering: Fundamentals, Devices, Fabrication, and Applications, Volume 5, 2006
(16) EP-A-1 992 404
(17) EP-A-2 017 000

## Claims

1. A process for oxidation of at least one alcohol group of at least one chemical compound to the corresponding carbonyl group, in the presence of a buffered oxidative hypohalous solution and of a nitroxide oxidation catalyst, wherein it is carried out within a micro-reactor, the reactant passage(s) of which showing a circular or non-circular section having a hydraulic diameter within the range: 0.2- 15 mm; and wherein the buffered oxidative hypohalous solution is prepared at a buffered pH comprised between 7 and less than 8.5 (7 ≤ pH < 8.5), advantageously comprised between 7.5 and 8.4 (7.5 ≤ pH ≤ 8.4), and is used less than 10 minutes after its preparation.

2. The process according to claim 1 wherein it is carried out for the oxidation of primary alcohol group(s) and wherein the buffered oxidative hypohalous solution is prepared at a buffered pH between 7.8 and 8.2 (7.8 ≤ pH ≤ 8.2).

3. The process according to claim 1 wherein it is carried out for the oxidation of secondary alcohol group(s) and wherein the buffered oxidative hypohalous solution is prepared at a buffered pH between 8 and 8.4 (8 ≤ pH ≤8.4).

4. The process according to any one of claims 1 to 3 wherein the buffered oxidative hypohalous solution is used less than 3 minutes after its preparation.

5. The process according to any one of claims 1 to 4 wherein the buffered oxidative hypohalous solution is batchwise prepared outside the micro-reactor before its introduction into said micro-reactor.

6. The process according to any one of claims 1 to 4 wherein the buffered oxidative hypohalous solution is in-line prepared.

7. The process according to any one of claims 1 to 6 wherein it is carried out with a concentration of the catalyst less than 5 mol %, advantageously less than 2 mol %, very advantageously less than 0.5 mol %.

8. The process according to any one of claims 1 to 7 wherein it is carried out for the oxidation of primary alcohol group(s) with a concentration of the catalyst less than 2 mol %, advantageously less than 0.5 mol %, very advantageously less than 0.07 mol %.

9. The process according to any one of claims 1 to 8, wherein it is carried out without any co-catalyst.

10. The process according to any one of claims 1 to 9 wherein the oxidative buffered hypohalous solution is a bleach solution and/or the nitroxide oxidation catalyst is selected from the group consisting in the (2,2,6,6-tetramethylpiperidin-1-yl)oxyl (TEMPO), in the TEMPO derivatives, and in the TEMPO or TEMPO derivatives on a support.

11. The process according to any one of claims 1 to 10 wherein the chemical compound or at least one of the chemical compounds is injected within the micro-reactor at at least two injection points, at least one downstream from a first injection point.

12. The process according to any one of claims 1 to 11 wherein it is carried within a micro-reactor with at least two mixing zones along the reactant passage.

13. The process according to claim 12 wherein said reactant passage has, successively along its length, at least two inlets, at least one for the buffered oxidative hypohalous solution, and at least one for the chemical compound(s) and TEMPO containing solution, an initial mixer passage portion, an initial dwell time passage portion having a volume of at least 0.1 ml and one or more additional mixer passage portions, each additional mixer passage portion followed immediately by a corresponding respective additional dwell time passage portion.

14. The process according to any one of claims 1 to 11 wherein it is carried within a micro-reactor with permanent mixing all along the reactant passage.

15. The process according to claim 14 wherein said reactant passage comprises multiple successive chambers, each chamber including a split of the reactant passage into at least two sub-passages, and a joining of the split passages, and a change of passage direction, of at least one of the sub-passages, of at least 90 degrees.

16. The process according to any one of claims 1 to 15 wherein it is carried out for oxidation of the alcohol group or all the alcohol groups of a single chemical compound, wherein it is advantageously carried out for oxidation of a single alcohol group of a single chemical compound, wherein it is very advantageously carried out for oxidation of a single primary alcohol group of a single chemical compound.

17. The process according to any one of claims 1 to 15 wherein it is carried out for a selective oxidation of at least one alcohol group within a mixture comprising of at least two chemical compounds, each having at least one alcohol group or of at least one alcohol group of a single chemical compound.

18. The process according to claim 17 wherein it is carried out for the selective oxidation of primary alcohol group(s) within a mixture comprising of at least two chemical compounds, each one having at least one alcohol group, or for the selective oxidation of primary alcohol group(s) of a chemical compound having said primary alcohol group(s) and at least one secondary alcohol group, or for selective oxidation of at least one primary alcohol group of a chemical compound having primary alcohol groups or for selective oxidation of at least one secondary alcohol group of a chemical compound having secondary alcohol groups.

## Patentansprüche

1. Verfahren zur Oxidation wenigstens einer Alkoholgruppe wenigstens einer chemischen Verbindung in die entsprechende Carbonylgruppe in der Anwesenheit einer gepufferten oxidativen hypohalogenigen Lösung und eines Nitroxid-Oxidationskatalysators, wobei es in einem Mikroreaktor durchgeführt wird, dessen Reaktantendurchgang/-durchgänge einen runden oder einen nicht-runden Abschnitt aufweist/aufweisen, der/die einen hydraulischen Durchmesser im Bereich von 0,2 bis 15 mm aufweist/aufweisen, und wobei die gepufferte oxidative hypohalogenige Lösung bei einem gepufferten pH-Wert zwischen 7 und weniger als 8,5 (7 ≤ pH < 8,5), vorteilhafterweise zwischen 7,5 und 8,4 (7,5 ≤ pH ≤ 8,4), zubereitet wird und in weniger als 10 Minuten nach ihrer Zubereitung verwendet wird.

2. Verfahren nach Anspruch 1, wobei es zur Oxidation einer/von primären Alkoholgruppe/n durchgeführt wird und wobei die gepufferte oxidative hypohalogenige Lösung bei einem gepufferten pH-Wert zwischen 7,8 und 8,2 (7,8 ≤ pH ≤ 8,2) zubereitet wird.

3. Verfahren nach Anspruch 1, wobei es zur Oxidation einer/von sekundären Alkoholgruppe/n durchgeführt wird und wobei die gepufferte hypohalogenige Lösung bei einem gepufferten pH-Wert zwischen 8 und 8,4 (8 ≤ pH ≤ 8,4) zubereitet wird.

4. Verfahren nach einem der Ansprüche 1 bis 3, wobei die gepufferte oxidative hypohalogenige Lösung weniger als 3 Minuten nach ihrer Zubereitung verwendet wird.

5. Verfahren nach einem der Ansprüche 1 bis 4, wobei die gepufferte oxidative hypohalogenige Lösung vor ihrer Einleitung in den Mikroreaktor batchweise außerhalb des Mikroreaktors zubereitet wird.

6. Verfahren nach einem der Ansprüche 1 bis 4, wobei die gepufferte oxidative hypohalogenige Lösung in-line zubereitet wird.

7. Verfahren nach einem der Ansprüche 1 bis 6, wobei es mit einer Konzentration des Katalysators von weniger als 5 mol-%, vorteilhafterweise weniger als 2 mol-%, weiter vorteilhafterweise weniger als 0,5 mol-%, durchgeführt wird.

8. Verfahren nach einem der Ansprüche 1 bis 7, wobei es zur Oxidation einer/von primären Alkoholgruppe/n mit einer Konzentration des Katalysators von weniger als 2 mol-%, vorteilhafterweise weniger als 0,5 mol-%, weiter vorteilhafterweise weniger als 0,07 mol-%, durchgeführt wird.

9. Verfahren nach einem der Ansprüche 1 bis 8, wobei es ohne einen CoKatalysator durchgeführt wird.

10. Verfahren nach einem der Ansprüche 1 bis 9, wobei die gepufferte oxidative hypohalogenige Lösung eine Bleichlösung ist und/oder der Nitroxid-Oxidationskatalysator aus der aus (2,2,6,6-Tetramethylpiperidin-1-yl)-oxyl (TEMPO), TEMPO-Derivaten und TEMPO oder TEMPO-Derivaten auf einem Träger bestehenden Gruppe ausgewählt ist.

11. Verfahren nach einem der Ansprüche 1 bis 10, wobei die chemische Verbindung oder wenigstens eine der chemischen Verbindungen an wenigstens zwei Injektionspunkten in den Mikroreaktor injiziert wird, wobei wenigstens einer stromabwärts von einem ersten Injektionspunkt liegt.

12. Verfahren nach einem der Ansprüche 1 bis 11, wobei es in einem Mikroreaktor mit wenigstens zwei Mischzonen entlang des Reaktantendurchgangs durchgeführt wird.

13. Verfahren nach Anspruch 12, wobei der Reaktantendurchgang nacheinander entlang seiner Länge wenigstens zwei Einlässe, wobei wenigstens einer für die gepufferte oxidative hypohalogenige Lösung und wenigstens einer für die chemische/n Verbindung/en und die TEMPO-haltige Lösung vorgesehen sind, einen Anfangsmischdurchgangsabschnitt, einen Anfangsverweilzeitdurchgangsabschnitt mit einem Volumen von wenigstens 0,1 ml und einen oder mehrere zusätzliche/n Mischdurchgangsabschnitt/e, wobei auf jeden zusätzlichen Mischdurchgangsabschnitt unmittelbar ein entsprechender zusätzlicher Verweilzeitdurchgangsabschnitt folgt, aufweist.

14. Verfahren nach einem der Ansprüche 1 bis 11, wobei es in einem Mikroreaktor unter ständigem Rühren entlang des ganzen Reaktantendurchgangs durchgeführt wird.

15. Verfahren nach Anspruch 14, wobei der Reaktantendurchgang mehrere aufeinanderfolgende Kammern umfasst, wobei jede Kammer ein Aufspalten des Reaktantendurchgangs in wenigstens zwei Unterdurchgänge und ein Verbinden der aufgespaltenen Durchgänge und eine Änderung der Durchgangsrichtung von wenigstens einem der Unterdurchgänge um wenigstens 90 Grad umfasst.

16. Verfahren nach einem der Ansprüche 1 bis 15, wobei es zur Oxidation der Alkoholgruppe oder aller Alkoholgruppen einer einzelnen chemischen Verbindung durchgeführt wird, wobei es vorteilhafterweise zur Oxidation einer einzelnen Alkoholgruppe einer einzelnen chemischen Verbindung durchgeführt wird, wobei es weiter vorteilhafterweise zur Oxidation einer einzelnen primären Alkoholgruppe einer einzelnen chemischen Verbindung durchgeführt wird.

17. Verfahren nach einem der Ansprüche 1 bis 15, wobei es für eine selektive Oxidation wenigstens einer Alkoholgruppe in einer Mischung, die wenigstens zwei chemische Verbindungen umfasst, von denen jede wenigstens eine Alkoholgruppe aufweist, oder wenigstens einer Alkoholgruppe einer einzelnen chemischen Verbindung durchgeführt wird.

18. Verfahren nach Anspruch 17, wobei es zur selektiven Oxidation einer/von primären Alkoholgruppe/n in einer Mischung, die wenigstens zwei chemische Verbindungen umfasst, von denen jede wenigstens eine Alkoholgruppe aufweist, oder zur selektiven Oxidation einer/von primären Alkoholgruppe/n einer chemischen Verbindung, die die primäre/n Alkoholgruppe/n und wenigstens eine sekundäre Alkoholgruppe aufweist, oder zur selektiven Oxidation wenigstens einer primären Alkoholgruppe einer chemischen Verbindung, die primäre Alkoholgruppen aufweist, oder zur selektiven Oxidation wenigstens einer sekundären Alkoholgruppe einer chemischen Verbindung, die sekundäre Alkoholgruppen aufweist, durchgeführt wird.

## Revendications

1. Procédé pour l'oxydation d'au moins un groupe alcool d'au moins un composé chimique en le groupe carbonyle correspondant, en présence d'une solution hypohalogéneuse oxydante tamponnée et d'un catalyseur d'oxydation de nitroxyde, mis en oeuvre dans un micro-réacteur, dont le(les) passage(s) de réactif présente(nt) une section circulaire ou non circulaire ayant un diamètre hydraulique dans l'intervalle : 0,2 - 15 mm ; et dans lequel la solution hypohalogéneuse oxydante tamponnée est préparée à un pH tamponné compris entre 7 et moins de 8,5 (7 ≤ pH < 8,5), avantageusement compris entre 7,5 et 8,4 (7,5 ≤ pH ≤ 8,4), et est utilisée moins de 10 minutes après sa préparation.

2. Procédé selon la revendication 1, mis en oeuvre pour l'oxydation d'un(de) groupe(s) alcool(s) primaire(s) et dans lequel la solution hypohalogéneuse oxydante tamponnée est préparée à un pH tamponné entre 7,8 et 8,2 (7,8 ≤ pH ≤ 8,2).

3. Procédé selon la revendication 1, mis en oeuvre pour l'oxydation d'un(de) groupe(s) alcool(s) secondaire(s) et dans lequel la solution hypohalogéneuse oxydante tamponnée est préparée à un pH tamponné entre 8 et 8,4 (8 ≤ pH ≤ 8,4).

4. Procédé selon l'une quelconque des revendications 1 à 3, dans lequel la solution hypohalogéneuse oxydante tamponnée est utilisée moins de 3 minutes après sa préparation.

5. Procédé selon l'une quelconque des revendications 1 à 4, dans lequel la solution hypohalogéneuse oxydante tamponnée est préparée de manière discontinue à l'extérieur du micro-réacteur avant son introduction dans ledit micro-réacteur.

6. Procédé selon l'une quelconque des revendications 1 à 4, dans lequel la solution hypohalogéneuse oxydante tamponnée est préparée en ligne.

7. Procédé selon l'une quelconque des revendications 1 à 6, mis en oeuvre avec une concentration de catalyseur inférieure à 5 % en mole, avantageusement inférieure à 2 % en mole, très avantageusement inférieure à 0,5 % en mole.

8. Procédé selon l'une quelconque des revendications 1 à 7, mis en oeuvre pour l'oxydation d'un(de) groupe(s) alcool(s) primaire(s) avec une concentration de catalyseur inférieure à 2 % en mole, avantageusement inférieure à 0,5 % en mole, très avantageusement inférieure à 0,07 % en mole.

9. Procédé selon l'une quelconque des revendications 1 à 8, mis en oeuvre sans co-catalyseur.

10. Procédé selon l'une quelconque des revendications 1 à 9, dans lequel la solution hypohalogéneuse tamponnée oxydante est une solution d'eau de Javel et/ou le catalyseur d'oxydation de nitroxyde est choisi dans le groupe constitué du (2,2,6,6-tétraméthylpîpéridin-1-yl)oxyle (TEMPO), des dérivés de TEMPO, et de TEMPO ou des dérivés de TEMPO sur un support.

11. Procédé selon l'une quelconque des revendications 1 à 10, dans lequel le composé chimique ou au moins un des composés chimiques est injecté dans le micro-réacteur en au moins deux points d'injection, au moins un en aval d'un premier point d'injection.

12. Procédé selon l'une quelconque des revendications 1 à 11, mis en oeuvre dans un micro-réacteur avec au moins deux zones de mélange le long du passage de réactif.

13. Procédé selon la revendication 12, dans lequel ledit passage de réactif présente, successivement le long de sa longueur, au moins deux entrées, au moins une pour la solution hypohalogéneuse oxydante tamponnée et au moins une pour la solution contenant le(les) composé(s) chimique(s) et TEMPO, une portion de passage de mélange initiale, une portion de passage de séjour initiale présentant un volume d'au moins 0,1 ml et une ou plusieurs portions de passage de mélange supplémentaire, chaque portion de passage de mélange supplémentaire suivie immédiatement par une portion de passage de séjour supplémentaire respective correspondante.

14. Procédé selon l'une quelconque des revendications 1 à 11, mis en oeuvre dans un micro-réacteur avec un mélange permanent tout le long du passage de réactif.

15. Procédé selon la revendication 14, dans lequel ledit passage de réactif comprend de multiples chambres successives, chaque chambre incluant une séparation du passage de réactif en au moins deux sous-passages, et une jonction des passages séparés, et un changement de direction de passage, d'au moins un des sous-passages, d'au moins 90 degrés.

16. Procédé selon l'une quelconque des revendications 1 à 15, mis en oeuvre pour l'oxydation du groupe alcool ou de tous les groupes alcools d'un unique composé chimique, mis en oeuvre avantageusement pour l'oxydation d'un unique groupe alcool d'un unique composé chimique, mis en oeuvre très avantageusement pour l'oxydation d'un unique groupe alcool primaire d'un unique composé chimique.

17. Procédé selon l'une quelconque des revendications 1 à 15, mis en oeuvre pour une oxydation sélective d'au moins un groupe alcool dans un mélange comprenant au moins deux composés chimiques, ayant chacun au moins un groupe alcool ou d'au moins un groupe alcool d'un unique composé chimique.

18. Procédé selon la revendication 17, mis en oeuvre pour l'oxydation sélective d'un(de) groupe(s) alcool(s) primaire(s) dans un mélange comprenant au moins deux composés chimiques, chacun présentant au moins un groupe alcool, ou pour l'oxydation sélective d'un(de) groupe(s) alcool(s) primaire(s) d'un composé chimique présentant ledit(lesdits) groupe(s) alcool(s) primaire(s) et au moins un groupe d'alcool secondaire, ou pour l'oxydation sélective d'au moins un groupe alcool primaire d'un composé chimique présentant des groupes d'alcools primaires ou pour l'oxydation sélective d'au moins un groupe alcool secondaire d'un composé chimique présentant des groupes d'alcools secondaires.
